# FASCICULE DE BREVET EUROPEEN

(11) **EP 1 653 963 B1**
(45) Date de publication et mention de la délivrance du brevet: **27.12.2006**
(21) Numéro de dépôt: 04767691.1
(22) Date de dépôt: 16.07.2004
(51) Int. Cl.: A61K 31/506, A61K 47/40, A61K 9/14, A61K 9/08

(54) **COMPOSITION PHARMACEUTIQUE NASALE DE PIRIBEDIL**
NASALE PHARMAZEUTISCHE ZUSAMMENSETZUNG VON PIRIBEDIL
NASAL PHARMACEUTICAL PIRIBEDIL COMPOSITION

(30) Priorité: 17.07.2003 FR 0308712
(43) Date de publication de la demande: 10.05.2006
(73) Titulaire: Les Laboratoires Servier, 92415 Courbevoie Cedex (FR)
(72) Inventeur: ROLLAND, Hervé, F-45160 Olivet (FR); WUTHRICH, Patrick, F-45000 Orléans (FR)
(86) Numéro de dépôt international: PCT/FR2004/001867
(87) Numéro de publication internationale: WO 2005/009442

(56) Documents cités:
- EP-A- 0 865 789
- FR-A- 2 827 516
- US-A- 5 955 454
- HAGUENAUER J P: "[Trivastan in otorhinolaryngology]" MINERVA MEDICA. ITALY 26 MAY 1976, vol. 67, no. 26, 26 mai 1976 (1976-05-26), pages 1698-1702, XP009028937 ISSN: 0026-4806

## Description

La présente invention concerne une composition pharmaceutique pour l'administration nasale de Piribédil.

Le Piribédil est un agoniste dopaminergique qui stimule les récepteurs à la dopamine et les voies dopaminergiques cérébrales et périphériques.

Le Piribédil est jusqu'alors administré par voie orale sous forme de comprimés à libération prolongée à avaler avec un demi-verre d'eau. Ces comprimés de Piribédil sont utiles pour le traitement du déficit pathologique cognitif et neurosensoriel chronique du sujet âgé, pour le traitement d'appoint de la claudication intermittente des artériopathies chroniques oblitérantes des membres inférieurs et dans le traitement de la maladie de Parkinson.

Le Piribédil peut également être administré par voie injectable afin d'améliorer les manifestations douloureuses des artériopathies en poussée ischémique, en association éventuellement avec un traitement chirurgical.

Des études pharmacocinétiques chez l'homme ont montré que la biodisponibilité du Piribédil par voie orale est faible par rapport à la voie parentérale et varie considérablement pour un même individu et d'un individu à l'autre.

La forme actuellement commercialisée de Piribédil est une forme à libération prolongée permettant l'absorption et la libération progressive du principe actif. A la dose de 50 mg, les études de cinétique chez l'homme ont montré un étalement de la couverture thérapeutique qui dépasse la durée du nycthémère.

Or, pour le traitement de la maladie de Parkinson notamment, la biodisponibilité faible du Piribédil ainsi que les variations des concentrations inter et intra-individuelles ont conduit à rechercher une nouvelle formulation permettant de remédier à ces inconvénients. D'autre part, il était particulièrement intéressant pour ces malades parkinsoniens qu'une forme à action rapide soit mise à la disposition du corps médical pour traiter les épisodes aigus très fréquents chez ces patients notamment pour la levée rapide de l'akinésie.

Les compositions pharmaceutiques de la présente invention permettent non seulement de remédier aux inconvénients connus de la forme à libération prolongée mais également de proposer un service médical rendu supérieur permettant notamment l'amélioration de la qualité de vie des patients. La muqueuse nasale largement vascularisée est particulièrement adaptée à l'absorption rapide du Piribédil pourvu que la forme pharmaceutique soit adaptée aux caractéristiques de ce principe actif.

Plus particulièrement, les compositions pharmaceutiques selon l'invention sont caractérisées en ce qu'elles contiennent le Piribédil ou l'un de ses sels pharmaceutiquement acceptables, éventuellement une cyclodextrine et un ou plusieurs excipients pharmaceutiquement acceptables.

Les compositions pharmaceutiques selon l'invention se présentent sous la forme de solutions aqueuses ou de poudres administrables à l'homme à l'aide d'un dispositif approprié permettant de délivrer à chaque pulvérisation la quantité de Piribédil nécessaire pour obtenir l'effet thérapeutique approprié.

Dans les compositions pharmaceutiques selon l'invention, le Piribédil se présente sous forme base ou d'un sel pharmaceutiquement acceptable.

Le Piribédil sera préférentiellement utilisé sous la forme base.

Les cyclodextrines utilisables dans les compositions pharmaceutiques selon l'invention sont plus spécifiquement les β-cyclodextrines. Parmi les β-cyclodextrines, on peut citer les β-cyclodextrines méthylées ou partiellement méthylées, l'hydroxypropyl-β-cyclodextrine, ou la sulfobutyléther-β-cyclodextrine. Les cyclodextrines préférées sont les cyclodextrines partiellement méthylées et de manière randomisée. La cyclodextrine partiellement méthylée et randomisée est préférentiellement la cyclodextrine dont le degré de substitution par des groupements méthyles est voisin de 1,7 (RAMEB). Dans les solutions nasales, les cyclodextrines seront préférentiellement ajoutées.

Dans les compositions pharmaceutiques en solution selon l'invention, la quantité de Piribédil (équivalent base) varie de 10 à 500 mg préférentiellement de 100 à 400 mg, la quantité de cyclodextrine varie de 75 à 3750 mg préférentiellement de 750 à 3000 mg pour une solution aqueuse finale de 10 ml.

Préférentiellement, pour une solution aqueuse finale de 10 ml, la quantité de Piribédil (équivalent base) est égale à 100 mg et la quantité de cyclodextrine partiellement méthylée (RAMEB) est égale à 750 mg.

Les solutions aqueuses pourront être rendues isotoniques par addition de chlorure de sodium par exemple. Le pH des solutions aqueuses sera préférentiellement ajusté à 6 par addition d'acide chlorhydrique.

Dans les compositions pharmaceutiques en poudre selon l'invention, la quantité de Piribédil varie de 0,1 à 20 mg préférentiellement de 1 à 10 mg, la quantité de cyclodextrine varie de 7,5 à 75 mg.

Les études cliniques réalisées chez des malades parkinsoniens avec les compositions pharmaceutiques selon l'invention ont montré une excellente tolérance chez l'homme, une meilleure biodisponibilité et une efficacité accrue par rapport à la forme orale actuellement commercialisée.

Les exemples suivants illustrent l'invention.

### EXEMPLE 1 :

Formulation solution :

| | |
|---|---|
| Piribédil base | 100 mg |
| RAMEB | 750 mg |
| Chlorure de sodium | 68 mg |
| Acide chlorhydrique 1Nqs | pH 6 |
| Eau purifiée qs | 10 ml |

Cette composition pharmaceutique est administrée au moyen d'une pompe doseuse délivrant 100 µl de solution, soit 1 mg de Piribédil base à chaque pulvérisation.

### EXEMPLE 2 :

Formulation solution :

| | |
|---|---|
| Piribédil base | 400 mg |
| RAMEB | 3000 mg |
| Chlorure de sodium | 65 mg |
| Acide chlorhydrique 1Nqs | pH 6 |
| Eau purifiée qs | 10 ml |

Cette composition pharmaceutique est administrée au moyen d'une pompe doseuse délivrant 100 µl de solution, soit 4 mg de Piribédil base à chaque pulvérisation.

### EXEMPLE 3 :

Formulation poudre :

| | |
|---|---|
| Piribédil base | 2 mg |
| RAMEB | 15 mg |
| Mannitol | 3 mg |

Cette composition pharmaceutique est administrée au moyen d'un spray-poudre délivrant 20 mg de poudre, soit 2 mg de Piribédil base à chaque pulvérisation.

### EXEMPLE 4 :

Formulation poudre :

| | |
|---|---|
| Piribédil base micronisée | 10 mg |
| Mannitol | 5 mg |

Cette composition pharmaceutique est administrée au moyen d'un spray-poudre délivrant 15 mg de poudre, soit 10 mg de Piribédil base à chaque pulvérisation.

### EXEMPLE 5:

Formulation poudre :

| | |
|---|---|
| Piribédil monomethane sulfonate | 2,65 mg |
| Lactose | 17,35 mg |

Cette composition pharmaceutique est administrée au moyen d'un spray-poudre délivrant 20 mg de poudre, soit 2 mg de Piribédil base à chaque pulvérisation.

### ETUDES CLINIQUES

### ETUDE DE CINETIQUE, DE TOLERANCE ET DE BIODISPONIBILITE CHEZ LE VOLONTAIRE SAIN

Une étude a été réalisée chez 24 volontaires sains afin d'évaluer la tolérance locale de la composition pharmaceutique selon l'invention ainsi que la cinétique de cette formulation.

Cette étude a été réalisée avec la formulation décrite dans l'exemple 1 administrée au moyen d'une pompe doseuse délivrant 100 µl de solution à chaque pulvérisation. Les doses de Piribédil testées sont les suivantes : 0,1 mg, 0,25 mg, 0,5 mg, 1 mg et 2 mg. Elles ont été administrées au moyen de deux pulvérisations de 100 µl chacune.

Cette étude a permis de montrer que la tolérance locale de la composition pharmaceutique selon l'invention est très bonne jusqu'à la dose de 2 mg. Les résultats des paramètres de cinétique ont montré que :
- La concentration maximale (C max) à la dose de 2 mg est d'environ 14 ng/ml. Cette dose correspond à la concentration plasmatique minimale efficace observée pour obtenir un effet thérapeutique sur les tremblements des malades parkinsoniens lorsque ceux-ci sont traités par voie injectable.
- Cette concentration maximale est obtenue 15 à 25 minutes après l'administration.
- Ces résultats ont permis de déduire que la biodisponibilité du Piribédil administré par voie nasale est comprise entre 50 et 70 %.

## Revendications

1. Composition pharmaceutique sous forme de solution aqueuse ou de poudre pour l'administration nasale de Piribédil **caractérisée en ce qu'**elle contient :
- du Piribédil ou un de ses sels pharmaceutiquement acceptables,
- éventuellement une cyclodextrine,
- un ou plusieurs excipients pharmaceutiquement acceptables.

2. Composition pharmaceutique selon la revendication 1 **caractérisée en ce que** le Piribédil se présente sous forme de base.

3. Composition pharmaceutique selon l'une quelconque des revendications 1 ou 2 **caractérisée en ce que** la cyclodextrine est une β-cyclodextrine partiellement méthylée.

4. Composition pharmaceutique selon la revendication 3 **caractérisée en ce que** la cyclodextrine est une β-cyclodextrine dont le degré de substitution par des groupements méthyles est voisin de 1,7.

5. Composition pharmaceutique selon l'une quelconque des revendications 1 à 4 **caractérisée en ce que** pour une solution aqueuse finale de 10 ml, la quantité de Piribédil est comprise entre 10 et 500 mg pour une quantité de cyclodextrine comprise entre 75 et 3750 mg.

6. Composition pharmaceutique selon l'une quelconque des revendications 1 à 4 **caractérisée en ce que** lorsque cette composition est sous forme de poudre, la quantité de Piribédil est comprise entre 0,1 mg et 20 mg pour une quantité de cyclodextrine comprise entre 7,5 et 75 mg.

## Claims

1. Pharmaceutical composition in the form of an aqueous solution or powder for the nasal administration of piribedil, **characterised in that** it comprises :
- piribedil or a pharmaceutically acceptable salt thereof,
- optionally a cyclodextrin,
- one or more pharmaceutically acceptable excipients.

2. Pharmaceutical composition according to claim 1, **characterised in that** the piribedil is in the form of the base.

3. Pharmaceutical composition according to either claim 1 or claim 2, **characterised in that** the cyclodextrin is a partially methylated β-cyclodextrin.

4. Pharmaceutical composition according to claim 3, **characterised in that** the cyclodextrin is a β-cyclodextrin wherein the degree of substitution by methyl groups is around 1.7.

5. Pharmaceutical composition according to any one of claims 1 to 4, **characterised in that**, for a final aqueous solution of 10 ml, the amount of piribedil is from 10 to 500 mg for an amount of cyclodextrin of from 75 to 3750 mg.

6. Pharmaceutical composition according to any one of claims 1 to 4, **characterised in that**, when the composition is in powder form, the amount of piribedil is from 0.1 mg to 20 mg for an amount of cyclodextrin of from 7.5 to 75 mg.

## Patentansprüche

1. Pharmazeutische Zubereitung in Form einer wäßrigen Lösung oder eines Pulvers für die nasale Verabreichung von Piribedil, **dadurch gekennzeichnet, daß** sie:
- Piribedil oder eines seiner pharmazeutisch annehmbaren Salze,
- gegebenenfalls ein Cyclodextrin,
- ein oder mehrere pharmazeutisch annehmbare Hilfsstoffe enthält.

2. Pharmazeutische Zubereitung nach Anspruch 1, **dadurch gekennzeichnet, daß** Piribedil in Form der Base vorliegt.

3. Pharmazeutische Zubereitung nach einem der Ansprüche 1 oder 2, **dadurch gekennzeichnet, daß** das Cyclodextrin ein teilweise methyliertes β-Cyclodextrin ist.

4. Pharmazeutische Zubereitung nach Anspruch 3, **dadurch gekennzeichnet, daß** das Cyclodextrin ein β-Cyclodextrin ist, dessen Methylgruppen-Substitutionsgrad etwa 1,7 beträgt.

5. Pharmazeutische Zubereitung nach einem der Ansprüche 1 bis 4, **dadurch gekennzeichnet, daß** für eine wäßrige Endlösung von 10 ml die Piribedil-Menge zwischen 10 und 500 mg beträgt, bei einer Cyclodextrinmenge zwischen 75 und 3750 mg.

6. Pharmazeutische Zubereitung nach einem der Ansprüche 1 bis 4, **dadurch gekennzeichnet, daß** für den Fall, daß diese Zubereitung in Form eines Pulvers vorliegt, die Piribedil-Menge zwischen 0,1 mg und 20 mg beträgt bei einer Cyclodextrin-Menge zwischen 7,5 und 75 mg.
